Europäisches Patentamt

European Patent Office

Office européen des brevets

(19)

(11) **EP 1 141 729 B1**

(12) **EUROPÄISCHE PATENTSCHRIFT**

(45) Veröffentlichungstag und Bekanntmachung des Hinweises auf die Patenterteilung:
**23.11.2005 Patentblatt 2005/47**

(51) Int Cl.[7]: **G01N 33/84**, G01N 31/22, G01N 33/18, G01N 21/64, G01N 21/77

(21) Anmeldenummer: **00900444.1**

(22) Anmeldetag: **12.01.2000**

(86) Internationale Anmeldenummer:
**PCT/AT2000/000004**

(87) Internationale Veröffentlichungsnummer:
**WO 2000/042438 (20.07.2000 Gazette 2000/29)**

(54) **OPTISCH-CHEMISCHER SENSOR ZUR BESTIMMUNG VON CHLORID**

OPTICAL-CHEMICAL SENSOR FOR DETECTING CHLORIDE

DETECTEUR OPTO-CHIMIQUE POUR LA DETERMINATION DES CHLORURES

(84) Benannte Vertragsstaaten:
**DE FR GB**

(30) Priorität: **12.01.1999 AT 3799**

(43) Veröffentlichungstag der Anmeldung:
**10.10.2001 Patentblatt 2001/41**

(73) Patentinhaber: **F. HOFFMANN-LA ROCHE AG**
**4070 Basel (CH)**

(72) Erfinder:
- **HUBER, Christian**
  **D-93326 Abensberg (DE)**
- **WERNER, Tobias**
  **D-93051 Regensburg (DE)**
- **WOLFBEIS, Otto, S.**
  **D-93051 Regensburg (DE)**
- **BELL, Douglas, E.**
  **Coral Springs, FL 33067 (US)**
- **YOUNG, Chi, Hui, Susannah, Tsai**
  **Norcross, GA 30092 (US)**

(74) Vertreter: **Schwarz, Albin et al**
**Patentanwalt**
**Wipplingerstrasse 32/22**
**1010 Wien (AT)**

(56) Entgegenhaltungen:
**AT-B- 384 891          US-A- 5 691 205**

- **SOUMILLION J -P ET AL: "A halogen anion sensor based on the hydrophobic entrapment of a fluorescent probe in silica sol-gel thin films" JOURNAL OF NON-CRYSTALLINE SOLIDS,NL,NORTH-HOLLAND PHYSICS PUBLISHING. AMSTERDAM, Bd. 220, Nr. 2-3, 1. November 1997 (1997-11-01), Seiten 316-322, XP004100618 ISSN: 0022-3093**
- **MARTIN A ET AL: "Studies on quenching of fluorescence of reagents in aqueous solution leading to an optical chloride-ion sensor" SENSORS AND ACTUATORS B,CH,ELSEVIER SEQUOIA S.A., LAUSANNE, Bd. 39, Nr. 1-3, 1. März 1997 (1997-03-01), Seiten 330-333, XP004087766 ISSN: 0925-4005**
- **BIWERSI JOACHIM; TULK BARRY; VERKMAN A S: "Long-wavelength chloride-sensitive fluorescent indicators" ANALYTICAL BIOCHEMISTRY, Bd. 219, 1994, Seiten 139-143, XP002135116**

## Beschreibung

Die Erfindung betrifft einen optisch-chemischen Sensor, welcher zur kontinuierlichen und diskontinuierlichen lumineszenzoptischen Bestimmung der Konzentration von Chlorid in einer wäßrigen Probe geeignet ist und welcher einen Lumineszenzindikator und ein den Lumineszenzindikator tragendes Polymer umfaßt, sowie ein Verfahren zur Herstellung eines solchen optisch-chemischen Sensors.

Chlorid ist die hauptsächliche anionische Substanz extrazellulärer Flüssigkeiten im Körper und spielt eine wichtige Rolle bei der Aufrechterhaltung des Verteilungsgleichgewichtes von Wasser, des osmotischen Druckes und des Gleichgewichtes anionischer und kationischer Bestandteile. Die Normalwerte für Chlorid im Blutserum des Menschen liegen bei 97 - 108 mmol/l, die Gesamtkonzentration anionischer Substanzen bei 154 mmol/l. Pathologische Chloridwerte - bis 170 mmol/l - finden sich bei osmotischer Diurese plus unzureichender Wasserzufuhr, bei gestörtem Mechanismus der Durstregulierung, nephrogenem Diabetes insipidus und enteralen Bicarbonatverlusten. Tiefstwerte - bis zu 30 mmol/l - finden sich bei gastrointestinalen Chloridverlusten und stehen im Zusammenhang mit der chronischen Pyelonephritis (Addison's disease) und Nierenversagen. Daher ist Chlorid in der klinischen Diagnostik das am häufigsten zu bestimmende Halogenid.

Neben titrimetrischen und photometrischen Bestimmungsverfahren, welche sehr genau, aber auch sehr arbeitsaufwendig sind, haben sich in der Praxis potentiometrische Verfahren mit ionenselektiven Elektroden durchgesetzt. Nachteilig ist die geringe Spezifität chloridselektiver Elektroden, ihre Empfindlichkeit gegenüber Proteinen und die Notwendigkeit einer Referenzelektrode.

In letzter Zeit wurde zur Bestimmung von Chlorid eine Reihe optischer Sensoren mit zum Teil unterschiedlich funktionierenden Indikatorsystemen vorgeschlagen. Die sogenannte Co-Extraktions-Optode (EP-0 358 991) beruht auf einem lipophilen pH-Indikatorfarbstoff mit lipophilem Gegenion, ist sehr empfindlich gegenüber lipophilen anionischen Bestandteilen der Probe und liefert nur bei sehr genau bekanntem pH-Wert brauchbare Ergebnisse.

Es ist bekannt, daß sowohl die Lumineszenzintensität als auch die Lumineszenzabklingzeit bestimmter Lumineszenzindikatoren durch dynamische Lumineszenzlöschung (Quenching) von Halogenidionen vermindert werden. Dies kommt durch die Stern-Vollmer-Gleichung zum Ausdruck:

$$\frac{F_0}{F} = 1 + k_q \cdot \tau_0 \cdot [Q] = 1 + K_{sv} \cdot [Q]$$

Hierin bedeuten $F_0$ und $F$ die relativen Lumineszenzintensitäten in Abwesenheit und Gegenwart eines Quenchers, [Q] die Konzentration des Quenchers, kq die bimolekulare Ratenkonstante für das Quenchen, $\tau_0$ die Lebenszeit des angeregten Zustands in Abwesenheit eines Quenchers und $K_{sv}$ die Stern-Vollmer-Quenchkonstante. Somit kann aus der relativen Lumineszenzintensität bzw. der Lumineszenzabklingzeit des Lumineszenzindikators auf die Chloridkonzentration der Probe geschlossen werden.

Eine chloridsensitive Optode auf der Grundlage eines kovalent an einer Glasoberfläche gebundenen Chinolin- oder Acridin-Farbstoffes, dessen Lumineszenz in Abhängigkeit der Halogenidkonzentration gelöscht wird, wurde in der AT-B 384 891 vorgeschlagen.

Chloridsensitive Lumineszenzindikatoren mit quaternierten heteroaromatischen N-Atomen weisen eine geringe pH-Querempfindlichkeit und geringe Querempfindlichkeit gegenüber physiologischen Konzentrationen an Störionen auf. Nachteilig für kommerzielle Anwendungen mit sehr hoher Stückzahl sind jedoch die im ultravioletten Spektralbereich liegenden Absorptionswellenlängen < 450 nm (mit kommerziell kostengünstig erhältlichen blauen LEDs nicht zugänglich) und die insbesondere in hoher Stückzahl aufwendige chemische Immobilisierung auf der Oberfläche geeigneter transparenter Trägermaterialien.

Zur Untersuchung des Chloridtransports und von Regulationsmechanismen in isolierten Membran-Vesikeln, rekonstituierten Liposomen und lebenden Zellen und Geweben durch Lumineszenzmessung sind eine Reihe von Quinolin- und Acridinderivaten und Lucigenin (ein Bisacridin) kommerziell erhältlich (Richard P. Haughland, "Handbook of Fluorescent Probes and Research Chemicals", 6. Auflage, S. 577-579).

Ein chloridsensitiver optisch-chemischer Sensor auf der Grundlage eines in einer Polyacrylamidschicht vorliegenden 3,6-Bis-(dimethylamino)acridins mit einer bis zu 30 C-Atome aufweisenden lipophilen aliphatischen Kohlenwasserstoffkette ist in der US-A-5 691 205 beschrieben. Dieser Indikator kann mit einer blauen Lichtquelle (LED) bei 488 nm angeregt werden.

Die Herstellung dieses bekannten Sensors umfaßt das Erzeugen einer dünnen, aus Polyacrylamid bestehenden Membran oder Schicht am Ende einer Lichtleiterfaser, und zwar durch Photopolymerisation einer Monomerenlösung, bestehend aus Acrylamid-N,N'-methylenbis(acrylamid), Riboflavin und Ammoniumperoxodisulfat. Anschließend wird die Membran bzw. Schicht in eine Indikatorlösung getaucht, wobei der lipophile Indikator in die Membran bzw. Schicht diffundiert. Über Stabilitätseigenschaften dieses Sensors, insbesondere über die Auswaschbarkeit des Indikators bei längerem Kontakt mit Meßflüssigkeiten, wie etwa Blut, werden in der US-A-5 691 205 keine Angaben gemacht.

Nachteilig ist bei dem aus der US-A-5 691 205 bekannten Sensor, insbesondere in Hinblick auf die Fertigung hoher Stückzahlen konstanter Qualität, der Fertigungsschritt der Membran bzw. Schicht durch Photopolymerisation einer Monomerenlösung und die Belegung der Membran bzw. der Schicht mit Indikator. Dieser Schritt ist in Hinblick auf eine konstante Qualität der Sensoren sehr aufwendig.

Die vorliegende Erfindung stellt sich die Aufgabe, einen optisch-chemischen Sensor, welcher zur lumineszenzoptischen Bestimmung der Konzentration von Chlorid in einer wäßrigen Probe geeignet ist, zur Verfügung zu stellen, der die oben angeführten Nachteile nicht aufweist. Insbesondere soll der Sensor mit kommerziell erhältlichen LEDs (Anregungswellenlänge > 460 nm) anregbar, in sehr hoher Stückzahl kostengünstig und reproduzierbar fertigbar und vorzugsweise zur Bestimmung physiologischer Chloridkonzentrationen einsetzbar sein.

Die erfindungsgemäße Aufgabe wird bei einem optisch-chemischen Sensor, welcher zur kontinuierlichen und diskontinuierlichen lumineszenzoptischen Bestimmung der Konzentration von Chlorid in einer wäßrigen Probe geeignet ist und welcher einen Lumineszenzindikator und ein den Lumineszenzindikator tragendes Polymer umfaßt, dadurch gelöst, daß der Lumineszenzindikator eine nicht lipophile Acridin- oder Bisacridinverbindung und das Polymer ein in einem organischen Lösungsmittel lösliches linearkettiges hydrophiles Polymer ist. Der Begriff "linearkettig" soll zum Ausdruck bringen, daß das Polymer nicht quervenetzt ist.

Es liegt auf der Hand, daß das Polymer nicht wesentlich in der Probe, z.B. Blut, Meerwasser, salzhältige wäßrige Flüssigkeiten, löslich sein sollte.

Die Vorteile eines solchen Sensors zur Messung von Chlorid liegen

- in einem großen dynamischen Meßbereich, insbesondere im physiologisch relevanten Konzentrationsbereich an Chlorid,
- in der hohen Sensitivität,
- in der hohen Stabilität und Reproduzierbarkeit,
- in der hohen Selektivität für Chlorid und
- in geringer pH-Querempfindlichkeit im physiologisch relevanten pH-Wertbereich.

Bevorzugt ist die Acridin- oder Bisacridinverbindung aus einer Gruppe, umfassend Methylacridiniummethosulfat (MAC), 4-Nitrophenylbutylacridiniummethosulfat (NPBA), N,N'-Di-(3-sulfopropyl)-9,9-bisacridinium (SPBA), N,N'-Di-essigsäureethylester-9,9-bisacridinium (AEBA) und Lucigenin, gewählt.

Als Polymer bevorzugt sind ionenpermeable Multi-Blockcopolymere, welche Säureamid- und Nitril- und/oder Säureimid- und/oder Carboxylatgruppen enthalten.

Ein solches linearkettiges hydrophiles Polymer ist beispielsweise das kommerziell erhältliche Multi-Blockcopolymer HYPAN, erhältlich von HYMEDIX Int. Inc., Dayton, N.J., welches weiter unten noch näher dargestellt werden wird. Entscheidend ist, daß dieses Polymer in einem organischen Lösungsmittel, beispielsweise DMSO, gelöst werden kann, wobei das Lösungsmittel nach Auftragen der Lösung auf ein transparentes Trägermaterial ebenso einfach wieder abgedampft werden kann, so daß eine einfache und reproduzierbare Herstellung eines Sensors ermöglicht wird.

Erfindungsgemäß einsetzbare Multi-Blockcopolymere sind dadurch gekennzeichnet, daß jede Polymerkette aus mehreren Sequenzen von Einheiten mit hydrophilen Eigenschaften, z.B. unabhängig voneinander Säureamidgruppen, die auch mit hydrophilen Gruppen substituiert sein können, Säureimidgruppen, Carboxylatgruppen und mehreren Sequenzen mit Gruppen hoher kohäsiver Energie, z.B. Nitrilgruppen, besteht. Die Herstellung derartiger Polymere ist beispielsweise in der US-A-4 331 783 und der US-A-4 379 874 beschrieben.

Überraschenderweise wurde festgestellt, daß die oben genannten Acridin- und Bisacridinverbindungen beim Eintauchen des linearkettigen hydrophilen Polymers in eine Lösung dieser Verbindungen in das Polymer diffundieren und danach nur mehr langsam auswaschbar sind. Somit stellt die vorliegende Erfindung einen sehr stabilen Sensor zur Verfügung.

Von den oben angeführten Acridin- und Bisacridinverbindungen ist Lucigenin aufgrund folgender Eigenschaften bevorzugt zur Bestimmung physiologischer Chlorid-Konzentrationen in Blut, Serum und Plasma geeignet:

(a) hohe Quench-Konstante Ksv in Lösung ($K_{sv}$(Cl⁻) > 100 M$^{-1}$),
(b) Anregungsmaximum über 450 nm, so daß eine kostengünstige LED als Lichtquelle eingesetzt werden kann,
(c) große Stokes-Verschiebung,
(d) hohe Photostabilität,
(e) Lumineszenz-Quantenausbeute höher als 0,5 und
(f) kommerzielle Verfügbarkeit.

Gemäß einer bevorzugten Ausführungsform des erfindungsgemäßen Sensors ist das den Lumineszenzindikator tragende Polymer in Form eines Films auf einem transparenten Trägermaterial aufgebracht. Der Film weist hierbei vorzugsweise eine Dicke bis zu 20 µm auf. Wesentlich ist, daß der Film in wäßrigen Flüssigkeiten, d.h. in der Probe oder in den Kalibrierflüssigkeiten, nicht löslich ist.

Gemäß einer weiteren bevorzugten Ausführungsform ist das den Lumineszenzindikator tragende Polymer in Form feiner Partikel in einem Hydrogel-Film eingebettet, der auf einem transparenten Trägermaterial aufgebracht ist. Die Größe der Partikel kann hierbei bis zu 20 µm betragen und beträgt vorzugsweise < 1 µm. Mittels dieser besonders vorteilhaften Ausführungsform können große Mengen an Lumineszenzindikator tragendem Material kostengünstig in einem Fertigungsschritt hergestellt werden. Ein weiterer wesentlicher Vorteil besteht darin, daß große Mengen an Sensoren mit diesem Material hergestellt werden können, die besonders einheitliche Charakteristiken, wie Kennlinie und Lumineszenzindikator-Beladung, aufweisen.

Erfindungsgemäß verwendete Hydrogele sind in Wasser oder wäßrigen Salzlösungen unlösliche ionenpermeable hydrophile Polymere. Bevorzugt sollen sie in organischen Lösungsmitteln oder Lösungsmittelgemischen (z.B. EtOH/H$_2$O) löslich sein, in denen das den Lumineszenzindikator tragende Polymer unlöslich ist, so daß nach der Auftragung der Schicht das Lösungsmittel abgedampft werden kann. Bevorzugt verwendete Hydrogele sind beispielsweise die linearkettigen hydrophilen Polymere der Fa. Tyndale Plains-Hunter (siehe unten). Grundsätzlich ist es auch möglich, andere Hydrogele zu verwenden, z.B. quervenetzte Polymere, insbesondere Polyacrylamid, wobei die Monomerenlösung nach dem Auftragen polymerisiert wird.

Vorzugsweise ist auf dem sich auf dem transparenten Trägermaterial befindlichen Film bzw. Hydrogel-Film eine zusätzliche Schicht aufgebracht, die aus einem hydrophilen, ionenpermeablen Polymer aufgebaut ist und vorzugsweise Farbpigmente, insbesondere Schwarzpigmente, enthält. Diese zusätzliche Schicht bewirkt vorteilhaft eine optische Entkopplung des Sensors vom Meßmedium. Sie wirkt als ionenpermeable optische Isolierschicht.

Gemäß einer weiteren Ausführungsform ist das transparente Trägermaterial eine Lichtleiterfaser, wobei der den Lumineszenzindikator tragende Film an einer Lichtleiterfaser aufgebracht ist.

Die Erfindung betrifft auch ein Verfahren zur Herstellung eines optisch-chemischen Sensors, welcher zur kontinuierlichen und diskontinuierlichen lumineszenzoptischen Bestimmung der Konzentration von Chlorid in einer wäßrigen Probe geeignet ist und welcher einen Lumineszenzindikator und ein den Lumineszenzindikator tragendes Polymer umfaßt, dadurch gekennzeichnet, daß

- als Lumineszenzindikator ein nicht lipophiler Lumineszenzindikator auf Basis einer Acridin- oder Bisacridinverbindung eingesetzt wird und
- als Polymer ein in einem organischen Lösungsmittel lösliches linearkettiges hydrophiles Polymer eingesetzt wird, wobei
- eine Lösung des Lumineszenzindikators hergestellt wird und
- die Lösung mit dem linearkettigen hydrophilen Polymer in Kontakt gebracht wird, wobei der Lumineszenzindikator in das linearkettige hydrophile Polymer diffundiert.

Gemäß einer bevorzugten Ausführungsform wird der in das linearkettige hydrophile Polymer diffundierte Lumineszenzindikator durch Bestrahlen, vorzugsweise mit ultraviolettem bzw. blauem Licht (320 nm bis 490 nm), immobilisiert.

Bei Verwendung eines Multi-Blockcopolymers vom Typ HYPAN wurde überraschenderweise festgestellt, daß insbesondere Acridine und Bisacridine mit zumindest einer Methylgruppe am quaternierten N-Atom (z.B. MAC, Lucigenin) nach Bestrahlen mit blauem bzw. ultraviolettem Licht auch bei längerem Kontakt mit Meßflüssigkeiten nicht ausgewaschen werden. Diese Nicht-Auswaschbarkeit ist insbesondere bei kontinuierlich durchgeführten Messungen ("monitoring") und bei einer Mehrfachverwendung von Sensoren wesentlich.

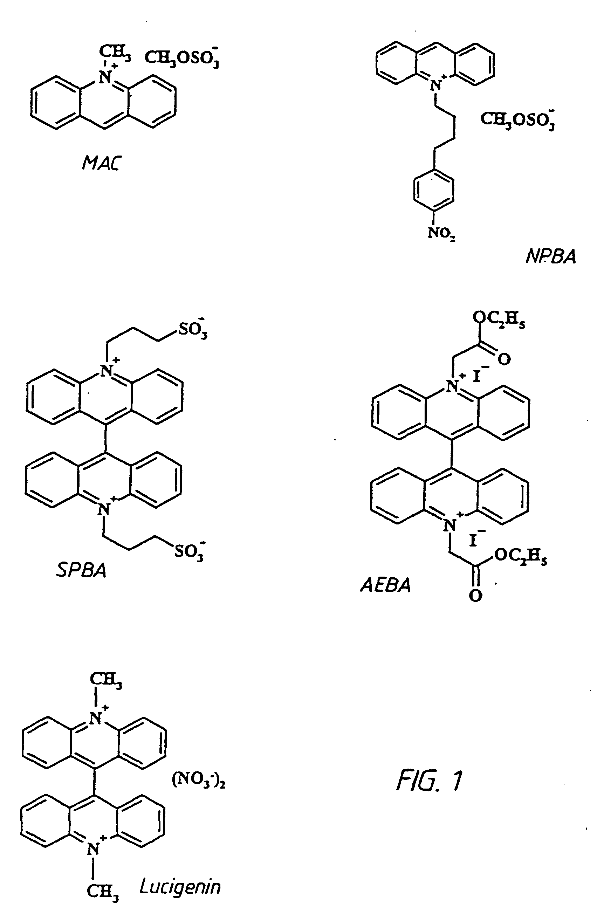

Versuche ergaben beispielsweise, daß mit blauem oder ultraviolettem Licht bestrahltes HYPAN HN80, welches MAC (Fig. 1) oder Lucigenin trug, nach Auflösen des Polymers in DMSO, Zentrifugieren der Lösung unter Zusatz von MeOH, das vom Polymer befreite Lösungsmittel klar war und die Polymerfraktion nach wie vor die typische Gelbfärbung des Indikators aufwies. Ein entsprechender Gegenversuch mit einem indikatortragenden Polymer, welches nicht mit blauem oder ultraviolettem Licht bestrahlt war, ergab, daß das vom Polymer befreite Lösungsmittel die typische Gelbfärbung des Indikators aufwies und die Polymerfraktion ungefärbt war. Dieser Versuch deutet darauf hin, daß die Bestrahlung des indikatortragenden Polymers mit blauem oder ultraviolettem Licht zu einer kovalenten Verknüpfung des Indikators mit dem Polymer führt, ohne dabei die Löschbarkeit der Indikatorlumineszenz nachteilig zu beeinflussen.

Versuche mit anderen Polymeren, welche Säureamidgruppen enthalten, z.B. Polyacrylamid, ergaben, daß sich die genannten Indikatoren ebenfalls durch Kontakt mit wäßrigen Indikatorlösungen in diese Polymere einbringen lassen, wonach sie langsam auswaschbar sind. MAC und Lucigenin werden nach Bestrahlung des Polymers mit blauem oder ultraviolettem Licht nicht mehr ausgewaschen.

Eine bevorzugte Ausführungsform des erfindungsgemäßen Verfahrens ist dadurch gekennzeichnet, daß

- eine Lösung des linearkettigen hydrophilen Polymers hergestellt wird,
- die Lösung auf ein transparentes Trägermaterial aufgebracht und das Lösungsmittel abdampfen gelassen wird, wobei ein Film des linearkettigen hydrophilen Polymers auf dem transparenten Trägermaterial gebildet wird,
- der Film mit der Lösung des Lumineszenzindikators in Kontakt gebracht wird, wobei der Lumineszenzindikator in den Film diffundiert und gegebenenfalls durch Bestrahlen immobilisiert wird.

Die Dicke des Films beträgt vorteilhaft bis zu 20 μm. Durch Stanzen kleiner Scheiben aus dem beschichteten transparenten Trägermaterial werden individuelle Sensor-Elemente hergestellt. Dieser Verfahrensschritt ist an sich aus der Literatur bekannt (M.J.P.

Leiner Optical Sensors for in vitro Blood Gas Analysis, Sensors and Actuators B 29, (1995) 169-173).

Eine weitere bevorzugte Ausführungsform des erfindungsgemäßen Verfahrens ist dadurch gekennzeichnet, daß

- die Lösung des Lumineszenzindikators mit pulvrigem linearkettigem hydrophilem Polymer in Kontakt gebracht wird, wobei der Lumineszenzindikator in das linearkettige hydrophile Polymer diffundiert und gegebenenfalls durch Bestrahlen immobilisiert wird,
- das den Lumineszenzindikator tragende linearkettige hydrophile Polymer in einer Hydrogellösung aufgeschlämmt wird, wobei eine Suspension gebildet wird,
- die Suspension auf ein transparentes Trägermaterial aufgebracht wird, und
- das Lösungsmittel aus der Hydrogellösung abdampfen gelassen wird, wobei auf dem transparenten Trägermaterial ein Hydrogel-Film gebildet wird, in dem das den Lumineszenzindikator tragende linearkettige hydrophile Polymer eingebettet ist.

Das linearkettige hydrophile Polymer wird hierbei bevorzugt mit einer Teilchengröße von kleiner als 1 $\mu$m eingesetzt. Die Teilchengröße kann aber auch bis 20 $\mu$m betragen.

Der Vorteil dieser Verfahrensweise liegt darin, daß durch Verwendung aliquoter Anteile ein- und derselben Charge des mit Lumineszenzindikator belegten pulvrigen linearkettigen hydrophilen Polymers für viele Sensor-Produktionschargen große Mengen an Sensoren mit sehr einheitlichen Eigenschaften hergestellt werden können.

Aus dem so beschichteten Trägermaterial können wiederum individuelle Sensor-Elemente durch Stanzen kleiner Scheiben hergestellt werden.

Die Erfindung betrifft weiters ein Verfahren zur Bestimmung der Konzentration von Chlorid in einer wäßrigen Probe unter Einsatz des erfindungsgemäßen optisch-chemischen Sensors.

Die Erfindung ist nachfolgend anhand der Zeichnung und anhand von Ausführungsbeispielen näher erläutert.

In Fig. 1 sind bevorzugt eingesetzte Lumineszenzindikatoren dargestellt. Die Figur zeigt die Strukturformeln der Lumineszenzindikatoren Methylacridiniummethosulfat(MAC),4-Nitrophenylbutylacridiniummethosulfat (NPBA), N,N'-Di-(3-sulfopropyl)-9,9-bisacridinium (SPBA), N,N'-Di-essigsäureethylester-9,9-bisacridinium (AEBA) und Lucigenin.

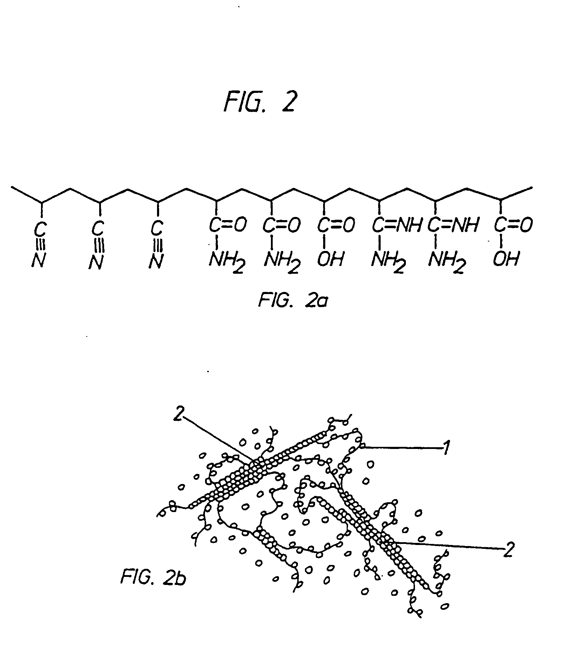

In Fig. 2 ist die Struktur eines bevorzugt eingesetzten linearkettigen hydrophilen Polymers dargestellt, welches unter dem Warenzeichen HYPAN (HYMEDIX Int. Inc., Dayton, N.J.) kommerziell erhältlich ist. Bei diesem Multi-Blockcopolymer bilden hydrophile Sequenzen durchgehende amorphe ionenpermeable Bereiche (sogenannte weiche Blöcke 1) aus, während kohäsive Sequenzen kristalline Cluster (sogenannte harte Blöcke 2) ausbilden. Eine genaue Beschreibung dieser Multi-Blockcopolymere findet sich in V.A. Stoy, New Type of Hydrogel for Controlled Drug Delivery, J. Biomedical Applications 3, 553-604 (1989).

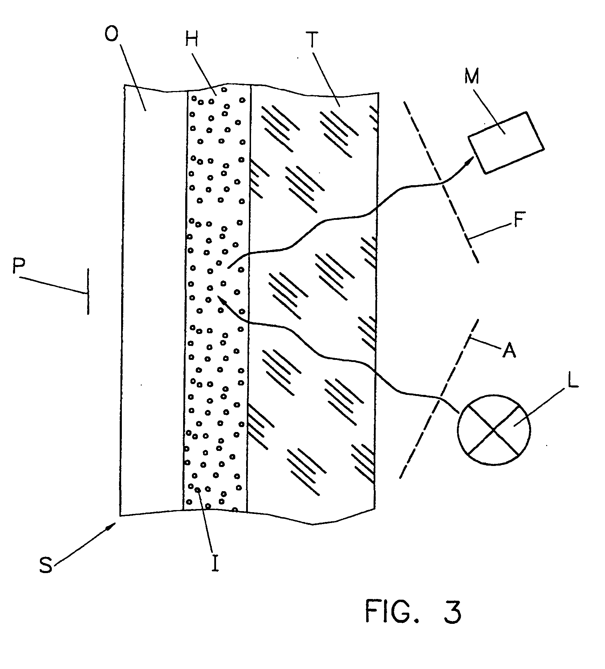

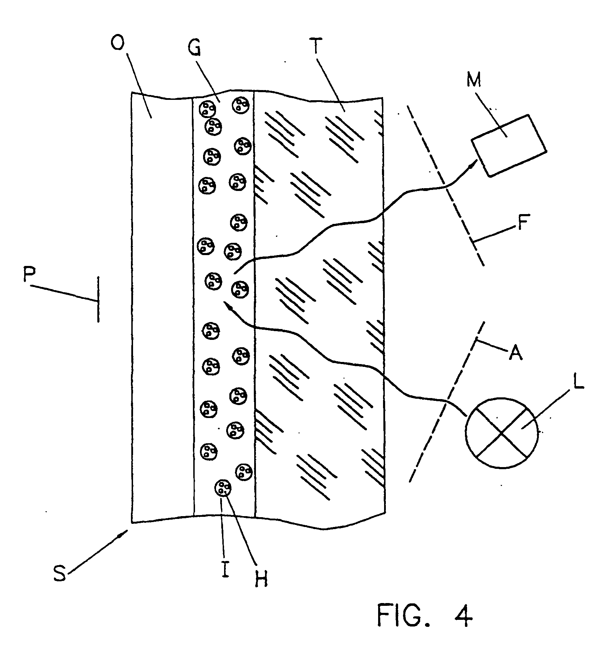

Fig. 3 zeigt schematisch den Aufbau eines erfindungsgemäßen Sensors gemäß einer bevorzugten Ausführungsform, wobei das den Lumineszenzindikator tragende Polymer in Form eines Films auf einem transparenten Trägermaterial aufgebracht ist. Fig. 4 zeigt schematisch eine weitere bevorzugte Ausführungsform des erfindungsgemäßen Sensors, wobei das den Lumineszenzindikator tragende Polymer in Form kleiner Partikel in einem Hydrogel-Film eingebettet ist, der auf dem transparenten Trägermaterial aufgebracht ist.

Die Fig. 3 und 4 zeigen jeweils eine Scheibe S des erfindungsgemäßen Sensors. Der in HYPAN immobilisierte chloridsensitive Lumineszenzindikator ist mit I bezeichnet. Gemäß Fig. 3 befindet sich das den Lumineszenzindikator I tragende HYPAN in Form eines dünnen Films H auf einem für das Anregungs- und Emissionslicht transparenten Trägermaterial T. Gemäß Fig. 4 befindet sich das den Lumineszenzindikator I tragende HYPAN in Form suspendierter Partikel in einem Hydrogel G, welches in Form eines dünnen Films auf einem für das Anregungs- und Emissionslicht transparentem Trägermaterial T aufgebracht ist. Zur optischen Entkopplung des den Lumineszenzindikator I tragenden Films vom Meßmedium P ist eine ionenpermeable optische Isolierschicht O, die aus einem pigmentierten Hydrogel besteht, auf dem Film H bzw. G aufgebracht.

Die optische Meßeinrichtung besteht aus einer LED als Lichtquelle L, einer Photodiode M als Detektor, optischen Filtern A und F zur Auswahl der Wellenlängen, einer optischen Anordnung zur Leitung des Anregungslichtes in die Polymerschicht H bzw. G und zur Leitung des Emissionslichtes zur Photodiode M sowie einer Einrichtung zur Signalverarbeitung (nicht dargestellt). Anregungsseitig wurde ein Interferenzfilter (Peak-Transmission bei 480 nm) und emissionsseitig ein 550 nm cut-off Filter verwendet.

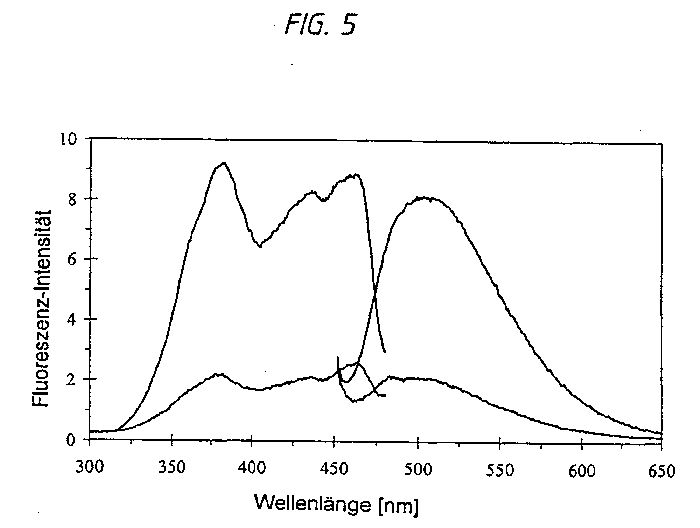

Fig. 5 zeigt das Anregungs- und Emissionsspektrum des beim erfindungsgemäßen Sensor bevorzugt eingesetzten Lumineszenzindikators Lucigenin, aufgenommen mit einer Meßanordnung gemäß Fig. 3 unter Verwendung eines handelsüblichen Fluorimeters.

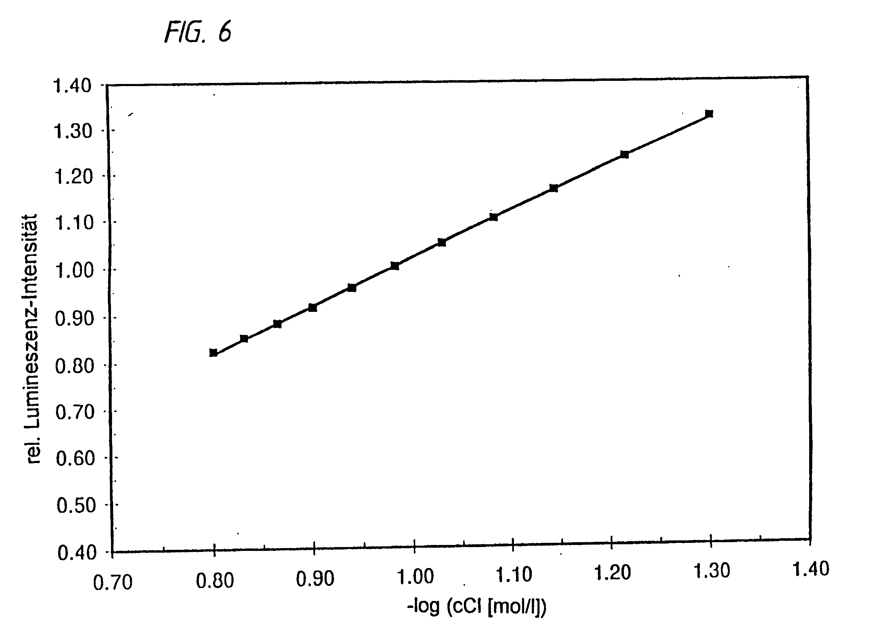

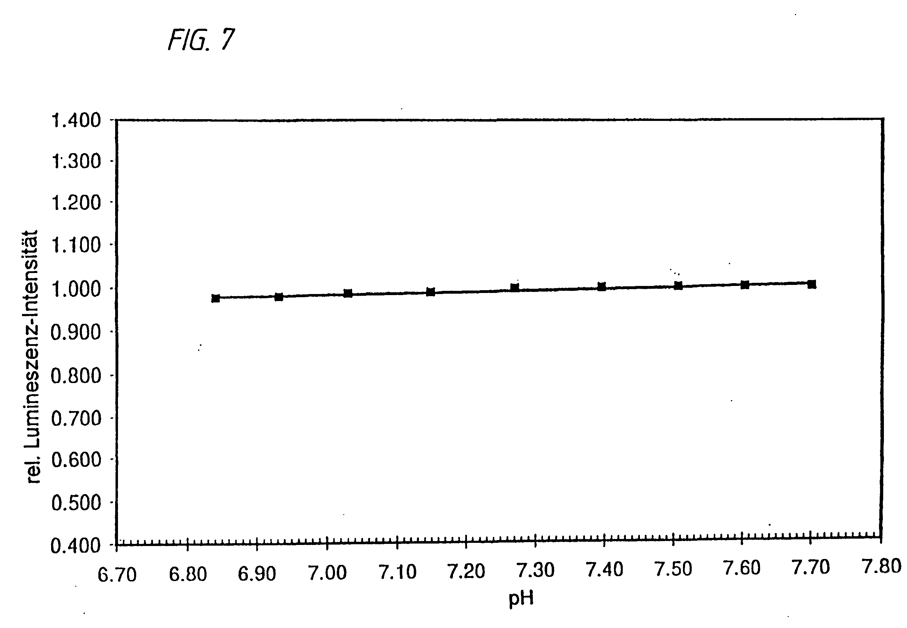

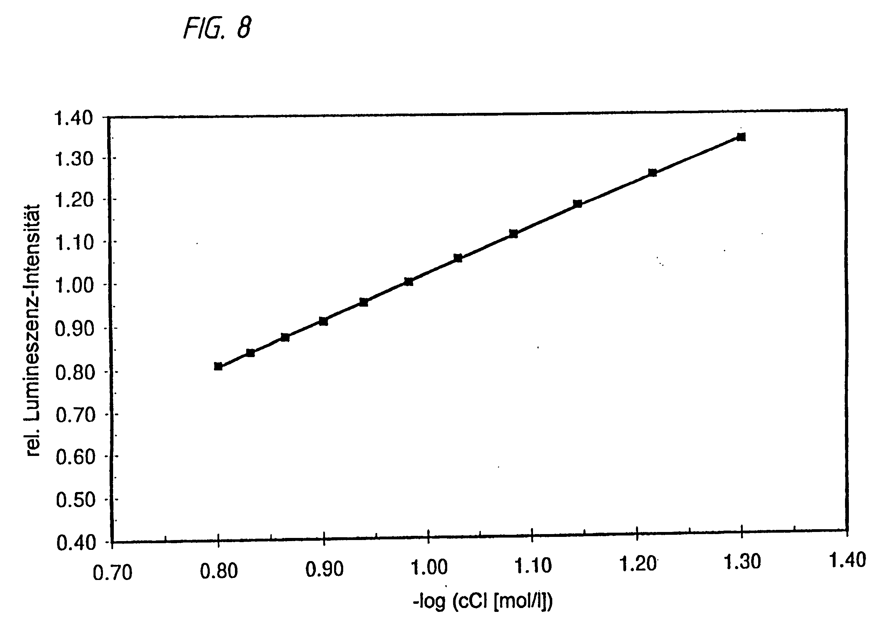

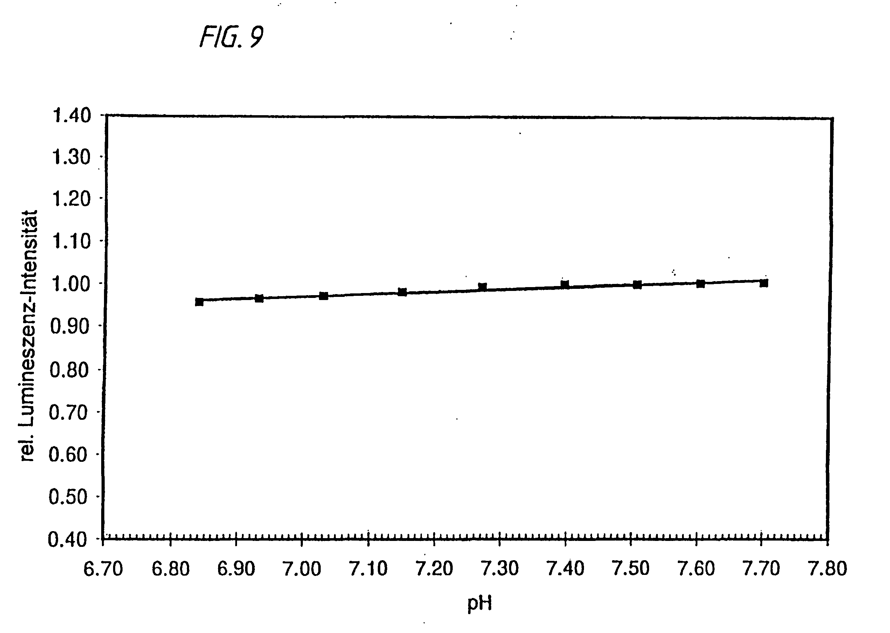

Fig. 6 zeigt relative Lumineszenzintensitäten eines erfindungsgemäßen Sensors nach dem Aufbauschema gemäß Fig. 3 in Abhängigkeit von der Konzentration an Chlorid. Fig. 7 zeigt relative Lumineszenzintensitäten dieses Sensors in Abhängigkeit vom pH-Wert des Meßmediums. Die Fig. 8 und 9 zeigen relative Lumineszenzintensitäten in Abhängigkeit von der Konzentration an Chlorid bzw. in Abhängigkeit vom pH-Wert für einen erfindungsgemäßen Sensor nach dem Aufbauschema gemäß Fig. 4.

Beispiel 1 (Fig. 3)

Herstellung von Sensoren mit einem den Lumineszenzindikator tragenden Polymerfilm

Herstellung von mit HYPAN beschichteten Folien:

6 g HYPAN HN80 (HYMEDIX Int. Inc., Dayton, N.J. 08810, USA) wurden mit Hilfe eines Magnetrührers in 80 ml DMSO bei 60° C über fünf Stunden aufgelöst. 4,5 ml der erhaltenen Lösung wurden auf eine auf einer planaren Unterlage befindliche Polyesterfolie (MYLAR, Plastic Suppliers, USA) mit Hilfe eines Haarlineals mit einer Naßdicke von 100 μm aufgetragen. Zwecks Abdampfens des Lösungsmittels wurde die Folie 20 Minuten bei Raumtemperatur belassen und anschließend 30 Sekunden in entionisiertes Wasser eingetaucht.

Belegen der mit HYPAN beschichteten Folien mit Lumineszenzindikator:

Eine 300 μM Lucigenin-Lösung (Aldrich Chemical Company Inc., # B4920-3, USA) wurde durch Auflösen von 135 mg Lucigenin in 1 Liter entionisiertem Wasser hergestellt. Anschließend wurde die HYPAN-beschichtete Folie in einem geeigneten Gefäß für 1 Stunde in die Lucigenin-Lösung eingetaucht, wobei ein Teil des Lucigenins in die HYPAN-Schicht der Folie diffundierte. Anschließend wurde die HYPAN-Schicht mit entionisiertem Wasser gewaschen.

UV-Behandlung der mit Lumineszenzindikator belegten Folie:

Zur Behandlung mit ultraviolettem Licht wurde die mit Lumineszenzindikator belegte Folie in einem offenen Gefäß mit der HYPAN-Schicht auf eine 12,7 cm (1/2 inch) dicke Schicht aus entionisiertem Wasser gelegt und anschließend eine Stunde aus einer Distanz von 10 cm mit einer UV-Lampe bestrahlt. Danach wurde die Folie zwecks Auswaschens von nichtgebundenem Indikator eine Stunde in 250 ml einer 100 mM NaF-Lösung (4,3 g NaF und 4,13 g $NaH_2PO_4$, gelöst in 900 ml entionisiertem Wasser, eingestellt mit 5 N NaOH auf pH 7,1 und anschließend mit $H_2O$ auf 1 Liter aufgefüllt) eingetaucht. Danach wurde die mit Lumineszenzindikator belegte HYPAN-Schicht mit entionisiertem Wasser gespült.

Herstellen und Aufbringen einer ionenpermeablen optischen Isolierschicht auf die mit Lumineszenzindikator belegte Folie:

1,5 g durch ein 25 μm Sieb gesiebte Cellulosefasern (VWR Scientific, P.O. Box 669967, Marietta, GA 30065, USA; Produkt Nr. JT 1528-1) und 3,5 g $Fe_2O_3$ (Fischer Scientific Company, # I116-3) wurden in 17 g einer Lösung, bestehend aus 1,8 Gew.% Hydrogel D6 (Tyndale Plains-Hunter Ltd., Ringoes, N.J. 08551, USA) in 55 Gew.% Ethanol in Wasser, 16 Stunden suspendiert. 5 ml der erhaltenen homogenen Suspension wurden mit einer Naßdicke von 100 μm auf die mit entionisiertem Wasser befeuchtete, mit Lumineszenzindikator belegte HYPAN-Schicht der UV-behandelten Folie aufgetragen. Nach dem Verdampfen des Lösungsmittels bei Raumtemperatur wurden Scheiben mit einem Durchmesser von 2,5 cm herausgeschnitten und bis zur Verwendung staubfrei und trocken bei Raumtemperatur gelagert. Vor der Verwendung wurden die Scheiben zur Aktivierung mindestens 16 Stunden im Puffer belassen.

Herstellung von Sensorscheiben:

Eine Methode zum Schneiden und Messen von Sensorscheiben wurde von M.J.P. Leiner und P. Hartmann in Sensors and Actuators B 11 (1993), Seiten 281 - 289 ("Theory and Practice in Optical pH Sensing") und von M.J.P. Leiner in Sensors and Actuators B 29 (1995), Seiten 169-173 ("Optical Sensors for in vitro Blood Gas Analysis") beschrieben.
Die gemäß dieser Methode erhaltenen Sensorscheiben wurden in der in der Fig. 3 schematisch dargestellten Meßanordnung verwendet.

Beispiel 2 (Fig. 4)

Herstellung von Sensoren aus mit Lumineszenzindikator belegten Partikeln

Herstellung von HYPAN-Partikeln:

Pulverförmiges HYPAN HN80 (mittlerer Korndurchmesser war 130 μm) wurde direkt vom Hersteller HYMEDIX bezogen. Kleinere Komdurchmesser (50 nm - 20 μm) wurden durch Feinmahlen des Materials mit einer Laborstrahlmühle (Glen Mills Inc., 395 Altwood Road, Cliftton, NJ 07012, USA) erhalten. Durch Sieben des feingemahlten Materials mit Metallsieben wurde eine Fraktion mit einem engeren Korndurchmesserbereich (50 - 500 nm) erhalten.

Belegen der HYPAN-Partikel mit Lumineszenzindikator und Immobilisieren des Indikators:

Eine 300 μM Lucigenin-Lösung wurde durch Auflösen von 135 mg Lucigenin in 1 Liter entionisiertem Wasser hergestellt und die Lösung in eine Kristallisierschale mit Magnetrührer gebracht. Anschließend wurden der Lösung langsam 50 g HYPAN-HN80-Pulver unter Rühren zugesetzt.
Eine UV-Lampe (Upland, CA 91786 USA, Model XX15, 115 V, 60 Hz, 0,68 A; Emission blau - nahes UV) wurde 10 cm über der Suspension positioniert und diese während 4 Stunden unter ständigem Rühren mit UV-Licht behandelt. Anschließend wurden die mit Indikator belegten Partikel mittels eines Büchnertrichters unter Verwendung eines Filterpapiers von der Belegungslösung getrennt, in eine weitere Kristallisierschale gebracht und dann 1 Stunde in 1 Liter einer 100 mM NaF-Lösung (4,3 NaF und 4,13 g $NaH_2PO_4$, gelöst in 900 ml entionisiertem Wasser, eingestellt mit 5 N NaOH auf pH 7,1 und anschließend mit $H_2O$ auf 1 Liter aufgefüllt) unter Rühren suspendiert. Die Suspension wurde filtriert und das Pulver mit entionisiertem Wasser gewaschen, bis das Filtrat farblos war. Anschließend wurde das Pulver im Büchnertrichter in absolutem Ethanol aufgeschlämmt und gewaschen und über Nacht bei Raumtemperatur getrocknet. Zwecks Entfernung von Restagglomeraten wurde das Pulver durch ein 50 µm Metallsieb gesiebt. Bis zur weiteren Verwendung wurde das Pulver in einem Exsikkator über $CaCl_2$ aufbewahrt.

Herstellen von mit Lumineszenzindikator beschichteten Folien:

1,2 g des auf 50 µm gesiebten indikatorbelegten Pulvers wurden 12 Stunden in 6,8 g einer Lösung, bestehend aus 1,8 Gew.% Hydrogel D6 (Tyndale Plains-Hunter Ltd., Ringoes, N.J.) in 55 Gew.% Ethanol in Wasser mit Hilfe eines Magnetrührers in einem geschlossenen Gefäß suspendiert. Anschließend wurde die homogene Suspension mit einer Naßdicke von 150 µm auf eine Polyesterfolie (MELINEX) aufgetragen und das Lösungsmittel über Nacht bei Raumtemperatur zum Verdampfen gebracht.

Das Aufbringen einer ionenpermeablen optischen Isolierschicht und die Herstellung von Sensorscheiben erfolgte wie unter Beispiel 1 beschrieben.

Bestimmung der Lumineszenzeigenschaften der erfindungsgemäßen Sensoren:

Zur Messung der Lumineszenzintensität erfindungsgemäßer Sensoren wurden gemäß oben beschriebenem Verfahren hergestellte Sensorscheiben in eine lichtdurchlässige thermostatisierte Meßzelle eingebracht und mit Proben, die unterschiedliche Chloridkonzentrationen und unterschiedliche pH-Werte aufwiesen, in Kontakt gebracht.

Fig. 5 zeigt das Lumineszenz-Anregungs- und -Emissionsspektrum eines nach Beispiel 1 hergestellten Sensors (ohne ionenpermeable optische Isolierschicht), bei pH 7,1, (A) in Kontakt mit einer 200 mM NaCl-Lösung und (B) in Kontakt mit einer 200 mM NaF-Lösung.

Die Fig. 6 bzw. 8 zeigen die relative Lumineszenzintensität erfindungsgemäßer Sensoren, hergestellt nach Beispiel 1 bzw. Beispiel 2, in Kontakt mit wäßrigen Lösungen in Abhängigkeit vom negativen dekadischen Logarithmus der Chloridkonzentration (0,050, 0,061, 0,072, 0,082, 0,093, 0,104, 0,115, 0,125, 0,136, 0,147, 0,159 mol/l).

Als Meßmedien wurden 0,1 M HEPES-Puffer, $CO_2$-frei, pH 7,4 (37°C) mit unterschiedlichen Konzentrationen an NaCl verwendet.

Wie aus den Fig. 6 und 8 ersichtlich ist, sind beide unterschiedlich aufgebauten Sensoren zur Bestimmung physiologischer (Blut-) Konzentrationen an Chlorid geeignet.

Die Fig. 7 bzw. 9 zeigen die relative Lumineszenzintensität erfindungsgemäßer Sensoren, hergestellt nach Beispiel 1 bzw. Beispiel 2, in Kontakt mit wäßrigen Lösungen in Abhängigkeit vom pH-Wert (6,819, 6,910, 7,009, 7,123, 7,250, 7,363, 7,481, 7,575, 7,671).

Als Meßmedien wurden 0,1 M HEPES-Puffer unterschiedlicher Konzentrationen an HEPES-Säure und HEPES-Na-Salz und mit einer Chlorid-Konzentration von 0,104 mol/l verwendet.

Wie aus den Fig. 7 und 9 ersichtlich ist, weisen die erfindungsgemäßen Sensoren im Bereich physiologischer pH-Werte keine Abhängigkeit vom pH-Wert des Meßmediums auf. Der leichte Anstieg der pH-Kennlinien ist auf eine schwache Wechselwirkung mit dem HEPES-Salz der beispielhaften Meßmedien zurückzuführen. Derartig hohe Konzentrationen an HEPES-Salz kommen in natürlichen Meßmedien (Körperflüssigkeiten, Meerwasser etc.) nicht vor.

Die vorliegende Erfindung ist nicht auf die dargestellten Ausführungsbeispiele beschränkt. Beispielsweise können vom Fachmann Sensoren unter Verwendung anderer chloridsensitiver Lumineszenzindikatoren als Lucigenin, etwa unter Verwendung der oben angeführten Verbindungen, auf analoge Weise hergestellt werden.

**Patentansprüche**

1. Optisch-chemischer Sensor, welcher zur kontinuierlichen und diskontinuierlichen lumineszenzoptischen Bestimmung der Konzentration von Chlorid in einer wäßrigen Probe geeignet ist und welcher einen Lumineszenzindikator (I) und ein den Lumineszenzindikator (I) tragendes Polymer (H) umfaßt, **dadurch gekennzeichnet, daß** der Lumineszenzindikator (I) eine nicht lipophile Acridin- oder Bisacridinverbindung und das Polymer (H) ein in einem organischen Lösungsmittel lösliches linearkettiges hydrophiles Polymer ist, wobei der Lumineszenzindikator (I) selbst über eine kovalente Verknüpfung im Polymer (H) immobilisiert vorliegt.

2. Optisch-chemischer Sensor nach Anspruch 1, **dadurch gekennzeichnet, daß** die Acridin- oder Bisacridinverbindung aus einer Gruppe, umfassend Methylacridiniummethosulfat (MAC), 4-Nitrophenylbutylacridiniummethosulfat (NPBA), N,N'-Di-(3-sulfopropyl)-9,9-bisacridinium (SPBA), N,N'-Di-essigsäureethylester-9,9-bisacridinium (AEBA) und Lucigenin, gewählt ist.

**3.** Optisch-chemischer Sensor nach Anspruch 1 oder 2, **dadurch gekennzeichnet, daß** das linearkettige hydrophile Polymer ein Multi-Blockcopolymer, enthaltend Säureamid- und/oder Säureimid- und/oder Carboxylat- und/oder Nitrilgruppen, ist.

**4.** Optisch-chemischer Sensor nach einem der Ansprüche 1 bis 3, **dadurch gekennzeichnet, daß** das den Lumineszenzindikator (I) tragende Polymer (H) in Form eines Films auf einem transparenten Trägermaterial (T) aufgebracht ist.

**5.** Optisch-chemischer Sensor nach einem der Ansprüche 1 bis 3, **dadurch gekennzeichnet, daß** das den Lumineszenzindikator (I) tragende Polymer (H) in Form feiner Partikel in einem Hydrogel-Film (G) eingebettet ist, der auf einem transparenten Trägermaterial (T) aufgebracht ist.

**6.** Optisch-chemischer Sensor nach Anspruch 4 oder 5, **dadurch gekennzeichnet, daß** auf dem sich auf dem transparenten Trägermaterial (T) befindlichen Film bzw. Hydrogel-Film (G) eine zusätzliche Schicht (O) aufgebracht ist, die aus einem hydrophilen ionenpermeablen Polymer aufgebaut ist und vorzugsweise Farbpigmente, insbesondere Schwarzpigmente, enthält.

**7.** Optisch-chemischer Sensor nach einem der Ansprüche 4 bis 6, **dadurch gekennzeichnet, daß** das transparente Trägermaterial (T) eine Lichtleiterfaser ist.

**8.** Verfahren zur Herstellung eines optisch-chemischen Sensors, welcher zur kontinuierlichen und diskontinuierlichen lumineszenzoptischen Bestimmung der Konzentration von Chlorid in einer wäßrigen Probe geeignet ist und welcher einen Lumineszenzindikator (I) und ein den Lumineszenzindikator (I) tragendes Polymer (H) umfaßt, **dadurch gekennzeichnet, daß**

- als Lumineszenzindikator (I) ein nicht lipophiler Lumineszenzindikator auf Basis einer Acridin- oder Bisacridinverbindung eingesetzt wird und
- als Polymer (H) ein in einem organischen Lösungsmittel lösliches linearkettiges hydrophiles Polymer eingesetzt wird, wobei
- eine Lösung des Lumineszenzindikators (I) hergestellt wird und
- die Lösung mit dem linearkettigen hydrophilen Polymer (H) in Kontakt gebracht wird,

wobei der Lumineszenzindikator (I) in das linearkettige hydrophile Polymer (H) diffundiert, und der in das linearkettige hydrophile Polymer (H) diffundierte Lumineszenzindikator (I) durch Bestrahlen immobilisiert wird.

**9.** Verfahren nach Anspruch 8, **dadurch gekennzeichnet, daß** der in das linearkettige hydrophile Polymer (H) diffundierte Lumineszenzindikator (I) durch Bestrahlen, mit ultraviolettem bzw. blauem Licht immobilisiert wird.

**10.** Verfahren nach Anspruch 8 oder 9, **dadurch gekennzeichnet, daß**

- eine Lösung des linearkettigen hydrophilen Polymers (H) hergestellt wird,
- die Lösung auf ein transparentes Trägermaterial (T) aufgebracht und das Lösungsmittel abdampfen gelassen wird, wobei ein Film des linearkettigen hydrophilen Polymers (H) auf dem transparenten Trägermaterial (T) gebildet wird,
- der Film mit der Lösung des Lumineszenzindikators (I) in Kontakt gebracht wird, wobei der Lumineszenzindikator (I) in den Film diffundiert und durch Bestrahlen immobilisiert wird.

**11.** Verfahren zur Herstellung eines optisch-chemischen Sensors, welcher zur kontinuierlichen und diskontinuierlichen lumineszenzoptischen Bestimmung der Konzentration von Chlorid in einer wäßrigen Probe geeignet ist und welcher einen Lumineszenzindikator (I) und ein den Lumineszenzindikator (I) tragendes Polymer (H) umfaßt, **dadurch gekennzeichnet, daß**

- eine Lösung des Lumineszenzindikators mit pulvrigem linearkettigem hydrophilem Polymer (H) in Kontakt gebracht wird, wobei der Lumineszenzindikator (I) in das linearkettige hydrophile Polymer (H) diffundiert und gegebenenfalls durch Bestrahlen immobilisiert wird,
- das den Lumineszenzindikator (I) tragende linearkettige hydrophile Polymer (H) in einer Hydrogellösung aufgeschlämmt wird, wobei eine Suspension gebildet wird,
- die Suspension auf ein transparentes Trägermaterial (T) aufgebracht wird und
- das Lösungsmittel aus der Hydrogellösung abdampfen gelassen wird, wobei auf dem transparenten Trägermaterial (T) ein Hydrogel-Film (G) gebildet wird, in dem das den Lumineszenzindikator (I) tragende linearkettige hydrophile Polymer (H) eingebettet ist.

**12.** Verfahren zur Bestimmung der Konzentration von Chlorid in einer wäßrigen Probe unter Einsatz eines optisch-chemischen Sensors nach einem der Ansprüche 1 bis 7.

**Claims**

**1.** An optical-chemical sensor which is suitable for the continuous and discontinuous determination by luminescence optics of the concentration of chloride in an aqueous sample and which comprises a luminescence indicator (I) and a polymer (H) carrying the luminescence indicator (I), **characterized in that** the luminescence indicator (I) is a non-lipophile acridine or bisacridine compound and the polymer (H) is a linear-chain hydrophile polymer soluble in an organic solvent, wherein the luminescence indicator (I) itself is provided immobilized in the polymer (H) via a covalent bond.

2. An optical-chemical sensor according to claim 1, **characterized in that** the acridine or bisacridine compound is selected from a group comprising methylacridinium methosulfate (MAC), 4-nitrophenylbutylacridinium methosulfate (NPBA), N,N'-di-(3-sulfopropyl)-9,9-bisacridinium (SPBA), N,N'-diacetic acid ethyl ester-9,9-bisacridinium (AEBA) and lucigenine.

3. An optical-chemical sensor according to claim 1 or 2, **characterized in that** the linear-chain hydrophile polymer is a multiple block copolymer containing acid amide and/or acid imide and/or carboxylate and/or nitrile groups.

4. An optical-chemical sensor according to any of claims 1 to 3, **characterized in that** the polymer (H) carrying the luminescence indicator (1) is applied in the form of a film to a transparent carrier material (T).

5. An optical-chemical sensor according to any of claims 1 to 3, **characterized in that** the polymer (H) carrying the luminescence indicator (I) is embedded in the form of fine particles in a hydrogel film (G) applied to a transparent carrier material (T).

6. An optical-chemical sensor according to claim 4 or 5, **characterized in that** an additional layer (O) is applied to the film or hydrogel film (G) that is on the transparent carrier material (T), which layer is composed of a hydrophile ion-permeable polymer and preferably contains color pigments, in particular black pigments.

7. An optical-chemical sensor according to any of claims 4 to 6, **characterized in that** the transparent carrier material (T) is a light-conducting fiber.

8. A process for the production of an optical-chemical sensor which is suitable for the continuous and discontinuous determination by luminescence optics of the concentration of chloride in an aqueous sample and which comprises a luminescence indicator (I) and a polymer (H) carrying the luminescence indicator (I), **characterized in that**

- a non-lipophile luminescence indicator on the basis of an acridine or bisacridine compound is used as luminescence indicator (I) and
- a linear-chain hydrophile polymer soluble in an organic solvent is used as polymer (H), wherein
- a solution of the luminescence indicator (I) is produced and
- the solution is brought into contact with the linear-chain hydrophile polymer (H), with the luminescence indicator (I) diffusing into the linear-chain hydrophile polymer (H), and the luminescence indicator (I) diffused into the linear-chain hydrophile polymer (H) is immobilized by radiation.

9. A process according to claim 8, **characterized in that** the luminescence indicator (I) diffused into the linear-chain hydrophile polymer (H) is immobilized by radiation with ultraviolet and/or blue light.

10. A process according to claim 8 or 9, **characterized in that**

- a solution of the linear-chain hydrophile polymer (H) is produced,
- the solution is applied to a transparent carrier material (T) and the solvent is allowed to evaporate, a film of the linear-chain hydrophile polymer (H) being formed on the transparent carrier material (T),
- the film is brought into contact with the solution of the luminescence indicator (I), the luminescence indicator (I) diffusing into the film and being immobilized by radiation.

11. A process for the manufacture of an optical-chemical sensor which is suitable for the continuous and discontinuous determination by luminescence optics of the concentration of chloride in an aqueous sample and which comprises a luminescence indicator (I) and a polymer (H) carrying the luminescence indicator (I), **characterized in that**

- a solution of the luminescence indicator is brought into contact with a powdery linear-chain hydrophile polymer (H), the luminescence indicator (I) diffusing into the linear-chain hydrophile polymer (H) and optionally being immobilized by radiation,
- the linear-chain hydrophile polymer (H) carrying the luminescence indicator (I) is suspended in a hydrogel solution, a suspension being formed,
- the suspension is applied to a transparent carrier material (T), and
- the solvent is allowed to evaporate from the hydrogel solution, wherein on the transparent carrier material (T) there is formed a hydrogel film (G) in which the linear-chain hydrophile polymer (H) carrying the luminescence indicator (I) is embedded.

12. A process for the determination of the concentration of chloride in an aqueous sample by using an optical-chemical sensor according to any of claims 1 to 7.

**Revendications**

1. Capteur opto-chimique approprié pour la détermination opto-luminescente continue et discontinue de la concentration de chlorure dans un échantillon aqueux et comprenant un indicateur de luminescence (I) et un polymère (H) portant l'indicateur de luminescence (I), **caractérisé en ce que** l'indicateur de luminescence (I) est un composé acridine ou bisacridine non lipophile et le polymère (H) est un polymère hydrophile à chaîne linéaire soluble dans un solvant organique, l'indicateur de luminescence se présentant lui-même immobilisé dans le polymère (H) par une liaison covalente.

2. Capteur opto-chimique selon la revendication 1, **caractérisé en ce que** le composé acridine ou bisacridine est choisi dans un groupe comprenant le méthosulfate de méthylacridinium (MAC), le méthosulfate de 4-nitrophénylbutylacridinium (NPBA), le N,N'-di-(3-sulfopropyl)-9,9-bisacridinium (SPBA), le N,N'-di-acide acétique-éthyl-ester-9,9-bisacridinium (AEBA) et la lucigénine.

3. Capteur opto-chimique selon la revendication 1 ou 2, **caractérisé en ce que** le polymère hydrophile à chaîne linéaire est un multi-copolymère bloc, contenant des groupes amide d'acide et/ou imide d'acide et/ou carboxylate et/ou nitrile.

4. Capteur opto-chimique selon l'une quelconque des revendications 1 à 3, **caractérisé en ce que** le polymère (H) portant l'indicateur de luminescence (I) est appliqué sous la forme d'un film sur un substrat transparent (T).

5. Capteur opto-chimique selon l'une quelconque des revendications 1 à 3, **caractérisé en ce que** le polymère (H) portant l'indicateur de luminescence (I) est incorporé sous forme de fines particules dans un film d'hydrogel (G) qui est appliqué sur un substrat (T).

6. Capteur opto-chimique selon la revendication 4 ou 5, **caractérisé en ce que** sur le film ou sur le film d'hydrogel (G) se trouvant sur le substrat (T) transparent est appliquée une couche supplémentaire (O) qui est constituée d'un polymère hydrophile perméable aux ions et contient de préférence des pigments colorés, en particulier des pigments noirs.

7. Capteur opto-chimique selon l'une quelconque des revendications 4 à 6, **caractérisé en ce que** le substrat transparent (T) est une fibre optique.

8. Procédé de production d'un capteur opto-chimique approprié pour la détermination opto-luminescente continue et discontinue de la concentration de chlorure dans une phase aqueuse et comprenant un indicateur de luminescence (I) et un polymère (H) portant un indicateur de luminescence (I), **caractérisé en ce que**

 - on utilise comme indicateur de luminescence (I) un indicateur de luminescence non lipophile à base d'un composé acridine ou bisacridine et
 - on utilise comme polymère (H) un polymère hydrophile à chaîne linéaire soluble dans un solvant organique,
 - une solution de l'indicateur de luminescence (I) est produite et
 - la solution est mise en contact avec le polymère hydrophile à chaîne linéaire (H), l'indicateur de luminescence (I) étant diffusé dans le polymère hydrophile à chaîne linéaire (H) et l'indicateur de luminescence (I) diffusé dans le polymère hydrophile à chaîne linéaire (H) étant immobilisé par rayonnement.

9. Procédé selon la revendication 8, **caractérisé en ce que** l'indicateur de luminescence (I) diffusé dans le polymère hydrophile à chaîne linéaire (H) est immobilisé par rayonnement au moyen d'une lumière ultraviolette ou d'une lumière bleue.

10. Procédé selon la revendication 8 ou 9, **caractérisé en ce que**

 - une solution du polymère hydrophile à chaîne linéaire (H) est produite,
 - la solution est placée sur un substrat (T) transparent et on élimine le solvant par évaporation, un film du polymère hydrophile à chaîne linéaire (H) étant formé sur le substrat transparent (T),
 - le film est mis en contact avec la solution de l'indicateur de luminescence (I), l'indicateur de luminescence (I) étant diffusé dans le film et immobilisé par rayonnement.

11. Procédé de fabrication d'un capteur opto-chimique approprié pour la détermination opto-luminescente continue et discontinue de la concentration de chlorure dans un échantillon aqueux et comprenant un indicateur de luminescence (I) et un polymère (H) portant l'indicateur de luminescence, **caractérisé**

**en ce que**

- une solution de l'indicateur de luminescence est mise en contact avec un polymère (H) hydrophile à chaîne linéaire pulvérulent, l'indicateur de luminescence (I) se diffusant dans le polymère hydrophile à chaîne linéaire (H) et étant éventuellement immobilisé par rayonnement,
- le polymère hydrophile à chaîne linéaire (H) portant l'indicateur de luminescence (I) est mis en suspension dans une solution d'hydrogel, une suspension étant formée,
- la suspension est placée sur un substrat transparent (T) et
- on élimine le solvant par évaporation de la solution d'hydrogel, un film d'hydrogel (G) étant formé sur le substrat transparent (T) dans lequel le polymère hydrophile à chaîne linéaire (H) portant l'indicateur de luminescence (I) est incorporé.

12. Procédé de détermination de la concentration de chlorure dans un échantillon aqueux en utilisant un capteur opto-chimique selon l'une quelconque des revendications 1 à 7.

MAC

NPBA

SPBA

AEBA

Lucigenin

FIG. 1

# FIG. 2

FIG. 2a

FIG. 2b

FIG. 3

FIG. 4

## FIG. 5

FIG. 6

FIG. 6

EP 1 141 729 B1

FIG. 7

FIG. 8

## FIG. 9